# EUROPÄISCHE PATENTSCHRIFT

(11) **EP 3 189 826 B1**
(45) Veröffentlichungstag und Bekanntmachung des Hinweises auf die Patenterteilung: **15.06.2022**
(21) Anmeldenummer: 16400062.2
(22) Anmeldetag: 28.12.2016
(51) Int. Cl.: A61K 6/818

(54) **KONDITIONIERUNG DER OBERFLÄCHE VON DENTALKOMPONENTEN**
CONDITIONING OF THE SURFACE OF DENTAL COMPONENTS
CONDITIONNEMENT DE LA SURFACE DE COMPOSANTS DENTAIRES

(30) Priorität: 29.12.2015 DE 102015017002
(43) Veröffentlichungstag der Anmeldung: 12.07.2017
(73) Patentinhaber: DCM Dental Creativ Management GmbH, 18055 Rostock (DE)
(72) Erfinder: Mitrovic, Milija, 18055 Rostock (DE)

(56) Entgegenhaltungen:
- DE-A1-102011 109 075
- DE-A1-102013 102 370
- DE-B3-102009 051 655

## Beschreibung

Die Erfindung betrifft Dentalkomponenten aus oxidkeramischen Werkstoffen auf der Basis von Zirkoniumdioxid (ZrO₂), dessen Mischkeramiken bzw. von Glaskeramiken mit Aufbissflächen für gegenüberliegende natürliche oder künstliche Zähne, die eine Konditionierung der Oberflächen aufweisen, wobei die Dentalkomponenten eine Beschichtung aus einem Glaslot aufweisen und einem keramischen Brand unterzogen werden.

In der Dentaltechnik werden bei der Herstellung von Dentalkomponenten aus oxidkeramischen Werkstoffen auf der Basis von Zirkoniumdioxid, dessen Mischkeramiken bzw. von Glaskeramiken, , die Gerüste für Kronen, Brücken oder andersgearteten Formteilen aus sogenannten "Weißlingen" gefertigt, d.h. aus Blöcken, die zuvor aus sogenannten "Grünlingen" gesintert wurden und die genügend Stabilität aufweisen, um daraus die Gerüste für einen Zahnersatz zu fräsen bzw., im Falle von Glaskeramiken, um diese presstechnisch herzustellen.

Probleme, die sich bei dieser Herstellungweise ergeben können, sind unter Umständen mangelnde Festigkeit und die Biokompatibiliät der bei derartigen Verfahren verwendeten keramischen Implantatwerkstoffe im Vergleich zu den üblicherweise verwendeten Implantaten aus Titanwerkstoffen. Insbesondere das Aufwachsen von Osteoblasten auf den Keramikoberflächen wird dadurch beeinträchtigt, woraus wiederum Haftungsprobleme resultieren können. Zur Funktionalisierung der so erzeugten Implantatoberflächen sind daher bereits verschiedene Verfahren zum Aufbringen von Titanbeschichtungen vorgeschlagen worden, um insbesondere die Haftung der Implantate zu verbessern.

Daneben ist in der DE 10 2013 102 370 A1 ein Verfahren der eingangs genannten Art beschrieben, bei dem ein keramisches Implantat mit einer Schicht belegt wird, vermittels derer es funktionalisiert und so biologisiert wird, dass seine Biokompatibilität zum umgebenden Knochengewebe verbessert und sein Einwachsen auf diese Weise beschleunigt wird.

Hierbei kann sich jedoch das Problem ergeben, dass so gefertigte Dentalkomponenten eine wesentlich größere Härte als die ihnen nach dem Einsetzen in den Oralbereich eines Patienten gegenüberstehenden natürlichen bzw. künstlichen Zähne aufweisen. In der Folge würden letztere auf Dauer dadurch zu stark beansprucht werden und so über Gebühr verschleißen, oder es würde die unkompensiert abgeleitete Kraft in andere biologische Strukturen abgeleitet werden und damit zu deren Überlastung und Schädigung führen. Insbesondere kann bei Glaskeramiken die Härte und Struktur der Keramikkompartimente zusätzlich zu einem Sandpapiereffekt führen.

Die DE 10 2013 102 370 A1 bezieht sich auf ein keramisches Implantat, wie bereits in der Beschreibungseinleitung diskutiert, dass belegt wird, vermittels derer es funktionalisiert und biologisiert wurde, dass seine Biokompalität zum umgebenden Knochengewebe verbessert und sein Einwachsen auf diese Weise beschleunigt wird.

Der Erfindung liegt die Aufgabe zugrunde, die Konditionierung der Oberfläche von Dentalkomponenten aus einem monolithisch konzipierten keramischen Zahnersatz auf der Basis von Zirkoniumdioxid, dessen Mischoxiden oder von Glaskeramiken, so auszubilden, dass eine signifikante Verbesserung der Aufbiss- und Gleiteigenschaften der Dentalkomponente ermöglicht wird.

Die Lösung der Aufgabe mit Bildung einer weichen Aufbissfläche zum allmählichen Abtrag erfolgt durch die Merkmale des Patentanspruches 1.

Dabei diffundiert die durch den keramischen Brand erzeugte glasige oder glaskeramische Schicht um einige Nanometer bei Oxidkeramiken bzw. um wenige Mikrometer im Falle von Glaskeramiken in die aus dichtgesintertem Zirkoniumdioxid bzw. dessen Mischoxiden oder aus Glaskeramik (LS2) bestehende Grenzschicht der Dentalkomponente ein. Dieser Vorgang ist sowohl bereits im Zusammenhang mit dem Verfahren der eingangs genannten Art beschrieben worden als auch in dem Artikel "Glasinfiltrierte Diffusionsschicht nach patentiertem Verfahren" der Firmenschrift "A Statement of Quality" der Firma DCM GmbH, Rev.3 vom 28.2.2013, wo unter anderem in der Abbildung "STEM Untersuchung" sowie auf der Seite mit der Überschrift "hotbond mono seal spray" das Hineindiffundieren der Komponenten der Glasmatrix in die Grenzschicht diskutiert wird.

Die Grenzschicht wird dabei weicher und es wird in der Folge weniger Stress durch die auftretenden Kaubelastungen auf die gegenüberstehenden natürlichen bzw. künstlichen Zähne ausgeübt. Durch die Härteerniedrigung infolge der spezifischen Zusammensetzung des Beschichtungsmaterials mit nur geringem bis fehlendem keramischen Anteil und sehr kleiner Partikelgröße wird so das einem Sandpapier ähnliche Abriebverhalten vermieden. Zugleich wird bei diesem keramischen Brand das aus dem silikatischen glaskeramischen Beschichtungswerkstoff bestehende Lot stoffschlüssig mit dem Substrat, d.h. mit der Dentalkomponente, verbunden und in ihr verankert. Dadurch wird zudem die Möglichkeit geschaffen, durch zusätzliches Aufbringen eines Stoffes zur Farbgebung die erzeugte Schicht vor dem abschließenden keramischen Brand einzufärben und die Dentalkomponente somit zu individualisieren.

Es hat sich in diesem Zusammenhang als vorteilhaft erwiesen, wenn die aus einem auf Siliziumdioxid (SiO₂) basierenden Glassystem bestehende Schicht mittels Sprühtechnik, insbesondere unter Verwendung einer sogenannten Airbrush-Pistole oder aus einer Sprayflasche, aufgebracht wird. Die dadurch erzeugte Dicke der Schicht liegt deutlich unter 20 Mikrometern und beeinflußt damit nicht die Passung der Prothetik gegenüber dem Antagonisten. Sie gewährleistet zugleich eine ausreichende Schichtstärke, um die auf diese Weise auf der Oberfläche erzeugte monolithische glasige/glaskeramische Schicht aus silikatischem Beschichtungswerkstoff anschließend in Bereichen okkusaler und approximaler Interferenzen weiter zu bearbeiten, ohne den beschriebenen Effekt aufzuheben.

Bei der Erfindung wird ein Keramiklot als Glaslot aus einem auf Siliziumdioxid-(SiO₂) basierendem biokompatiblem Glassystem verwendet, das nach dem keramischen Brand eine Härte von weniger als ein Bereich von 400 - 450 HV_{0,1} aufweist. Hierdurch ist eine Verbesserung der tribologischen Eigenschaften und damit ein Schutz der natürlichen Zahnsubstanz sowie gnathologisch relevanter benachbarter Gelenks-, Bänder- und Muskelstrukturen wie dem Kiefergelenk oder der Kau-, Nacken- und Halsmuskulatur gegeben.

Zwar ist es aus dem Artikel" Surface Modifications of Dental Ceramic Implants with Different Glass Solder Matrices: In Vitro Analyses with Human Primary Osteoblasts and Epithelial Cells" von J. Markhoff, E. Mick, A. Mitrovic, J. Pasold, K. Wegner und R. Bader in der Zeitschrift BioMed Research International, Vol. 2014., Article ID 742180,.http://dx.doi.org/10.1155/2014/742180, im Prinzip bereits bekannt, dass bei Bioglas-Coatings Teile des Glaslotes in das Keramiksubstrat hineindiffundieren und dessen Biegesteifigkeit herabsetzen können, jedoch gilt der Mechanismus dieses Vorganges bisher als nicht geklärt. So ist auch in der WO 2005/070322 A1 ein Verfahren zur Herstellung eines zunächst aus pulverförmig vorliegenden, Komponenten von Oxidkeramiken enthaltenden anorganisch-anorganischer Kompositwerkstoff für den Dentalbereich beschrieben, in den Bindemittel hineingesintert und damit die Biaxialfestigkeit des in diesem Fall als Ausgangswerkstoff verwendeten Komposits wesentlich erhöht werden kann, ohne dass die zugrunde liegenden Effekte vollends aufgeklärt werden könnten. Darüber hinaus ist es aus weiteren Artikeln, unter anderem in den Zeitschriften *Biomaterials* und *Biomedical Materials Research,* bereits bekannt, die Osseointegration von Dentalkomponenten mittels einer Beschichtung aus Bioglas zu verbessern. Eine Verwendung dieser Effekte im Sinne der vorliegenden Erfindung, d.h., zur Verbesserung der Aufbisseigenschaften einer Dentalkomponente, wird auch in diesen Artikeln jedoch nicht angedacht. Dies gilt schließlich auch für ein Verfahren zum elektrophoretischen Beschichten von keramischen Körpern unter anderem für dentale Zwecke, das aus der DE 699 25 725 T2 bekannt geworden ist.

Nachfolgend soll die Erfindung anhand der Zeichnung näher erläutert werden. Die Figuren 1 bis 3 zeigen dabei in einzelnen Schritten in schematischer Darstellung den Verfahrensablauf bei einer herzustellenden Verbindung, und zwar:
- Fig. 1: einen vertikalen Schnitt durch eine Dentalkomponente aus Zirkoniumdioxid nach ihrer Herstellung beispielsweise mittels eines CAD/CAM-Verfahrens,
- Fig. 2: einen Schnitt durch die Oberfläche gemäß Fig. 1 nach dem Aufbringen eines aus einem reaktiven glasigen oder glaskeramischen Beschichtungswerkstoff bestehenden silikatischen Lotes und
- Fig. 3: einen Schnitt durch die Oberfläche gemäß Fig. 2 nach dem keramischen Brand und einem anschließenden allmählichen Abtrag des silikatischen Lotes sowie von Teilen der Diffusionsschicht infolge primärer Bearbeitung oder langzeitiger Kaubeanspruchung.

In den Figuren sind gleiche Elemente mit den gleichen Bezugszeichen versehen.

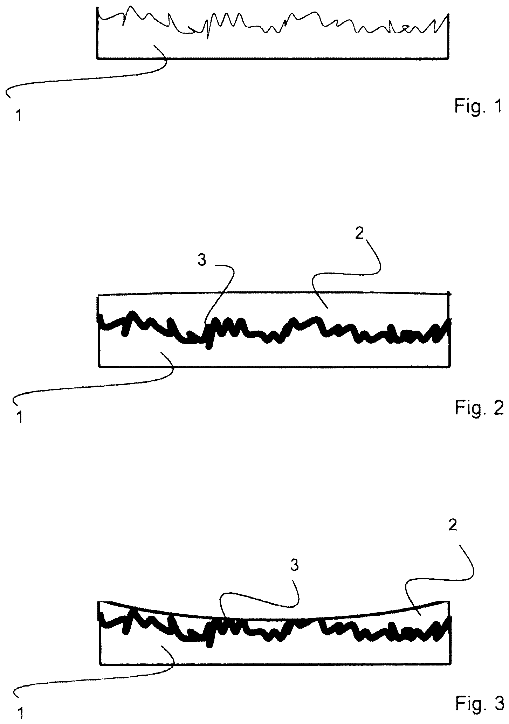

Gemäß dem in den Figuren dargestellten Verfahrensablauf wird die zu konditionierende Dentalkomponente 1 zunächst mit einem geeigneten Verfahren, beispielsweise mittels CAD/CAM, aus einem "Weißling", der aus einem oxidkeramischen Werkstoff auf der Basis von Zirkoniumdioxid, dessen Mischoxiden oder aus Glaskeramik (LS2) besteht, hergestellt und gegebenenfalls manuell nachbearbeitet. Die Oberfläche der Dentalkomponente 1 weist dann die im Vertikalschnitt in Fig. 1 dargestellte Struktur mit den durch den Herstellungsprozeß bedingten typischen Bearbeitungsspuren auf. Danach wird auf die Dentalkomponente 1 mittels Sprühtechnik mit einer Airbrush-Pistole bzw. aus einer Spraydose eine aus einem auf Siliziumdioxid (SiO₂) basierenden Glassystem bestehende Schicht 2 dünn aber gleichmäßig deckend aufgetragen und trocknen gelassen. Diese Schicht 2 bewirkt nach dem keramischen Brand nicht nur die Glättung der Kau- und Funktionsflächen, sondern sie kann bei Bedarf zugleich auch die gewünschte Einfärbung der sichtbaren Oberfläche der Dentalkomponente 1 gewährleisten.

Anschließend wird die auf diese Weise beschichtete Dentalkomponente 1 bei vorzugsweise etwa 700 bis 1100 °C einem keramischen Brand unterzogen, durch den sich eine Diffusionszone 3 von einigen Nanometern bis hin zu wenigen Mikrometern, abhängig vom beschichteten Material, ausbildet. Die Dicke der so neu gebildeten glasig/glaskeramischen Schicht 3 auf der Oberfläche der Dentalkomponente 1 beträgt dabei in der Regel weniger als 20 Mikrometer. Die Oberfläche der Dentalkomponente 1, bestehend aus der Schicht 2, ist zunächst sehr glatt und strukturlos. Die Bedeckung des versiegelten Bereiches ist fehlerfrei, das verwendete Glaslot auf der Basis eines auf Siliziumdioxid (SiO₂) basierenden Materials bewirkt eine gute Benetzung der Zirkondioxid-Oberfläche. Durch die definierte Dicke der Schicht ist eine geplante Strukturierung der Oberfläche aus phonetischer und ästhetischer Sicht, z.B. bei der Gestaltung von Frontzahnkronen (fehlender Speckglanz), möglich und auf Grund der physikalischen Eigenschaften der deckenden Schicht einfach und vorhersagbar.

Ein chemisches Anlösen des Zirkondioxids an den Rändern der Dentalkomponente 1 kann höchstens in der Dicke der Reaktionsschicht auftreten, wodurch aber die Kanten- und Formstabilität nicht beeinflusst wird. Das verwendete Glaslot ist bei der gewählten Verarbeitungstemperatur vollständig durchgeschmolzen und versiegelt dadurch die Zirkondioxid-Oberfläche gleichmäßig.

Die glasig/glaskeramische silikatische Schicht diffundiert bei diesem Prozess in die Grenzschicht der aus einem oxidkeramischen Werkstoff auf der Basis von Zirkoniumdioxid bestehenden Dentalkomponente 1 ein und macht sie damit zugleich weicher. Beim keramischen Brand wird außerdem das aus dem reaktiven biokompatiblen Glaslot aus einem auf Siliziumdioxid (SiO₂) basierende Glassystem stoffschlüssig mit dem darunter befindlichen Substrat, d.h. dem Zirkoniumdioxid im Bereich der Grenzschicht der Dentalkomponente 1, verankert.

Dadurch wird sichergestellt, dass der sichtbare Bereich der Oberfläche der Dentalkomponente 1 bei Kontakt mit einem natürlichen oder künstlichen Zahn bzw.

Zahnersatz als Aufbisspartner letzteren nachhaltig schont. Damit verbunden ist eine tribologische Optimierung durch die Konditionierung aller Funktionsoberflächen der betreffenden Dentalkomponente, unabhängig von der funktionellen Beanspruchung sowie den biologischen Herausforderungen im Bereich des Kronenrandes zum Anschluss an die Gingiva. Somit werden die extrem harten Dentalkomponenten aus Zirkoniumdioxid bzw. dessen Mischkeramiken der Härte natürlicher oder künstlicher Zähne angepasst und es wird als Schutz der natürlichen bzw. künstlichen Zahnsubstanz sowie der Gelenk- und anderer biologischer Strukturen der abrasive Verschleiß bzw. deren Überlastung reduziert.

Da die aus den "Weißlingen" gefertigten Dentalkomponenten nach dem Sintern noch rein weiß sind, können weiterhin ihre sichtbaren Oberflächen vor oder nach dem Sinterprozeß eingefärbt werden, so dass sichergestellt wird, dass der sichtbare Bereich der Dentalkomponente zugleich die angestrebte Farbgebung annimmt.

## Patentansprüche

1. Dentalkömponenten aus oxidkeramischen Werkstoffen auf der Basis von Zirkoniumdioxid (ZrO₂), dessen Mischkeramiken bzw. von Glaskeramiken mit Aufbissflächen für gegenüberliegende natürliche oder künstliche Zähne, die eine Konditionierung der Oberflächen aufweisen, wobei die Dentalkomponenten eine Beschichtung aus einem Glaslot aufweisen und einem keramischen Brand unterzogen werden und auf der sichtbaren Oberfläche der Dentalkomponente (1) im Bereich der Aufbissfläche abschließend eine Schicht (3) eines biokompatiblen Glaslotes aus einem auf Siliziumdioxid (SiO₂) basierenden Glassystem aufgetragen und einem keramischen Brand unterzogen wird, wobei die Dicke der silikatischen glaskeramischen Schicht (3) weniger als 20 Mikrometer beträgt und das Glassystem nach dem keramischen Brand eine Härte von weniger als ein Bereich von 400 - 450 HV_{0,1} aufweist.

2. Dentalkomponenten gemäß Anspruch 1, **dadurch gekennzeichnet, dass** das Glaslot mittels Sprühtechnik aufgebracht wird.

3. Dentalkomponenten gemäß Anspruch 1 oder 2, **dadurch gekennzeichnet, dass** das Glaslot einen Stoff zur Farbgebung enthält.

## Claims

1. Dental components produced from oxide ceramic substances based on zirconium dioxide (ZrO₂), mixed ceramics thereof or from glass ceramics with occlusal surfaces for opposing natural or prosthetic teeth which have surface conditioning, wherein the dental components have a coating produced from a glass solder and have undergone ceramic firing, and finally a layer (3) of a biocompatible glass solder produced from a glass system based on silicon dioxide (SiO₂) is applied to the visible surface of the dental component (1) in the region of the occlusal surface and undergoes ceramic firing, wherein the thickness of the silicate glass ceramic layer (3) is less than 20 micrometres and after ceramic firing, the glass system has a hardness below a range of 400 - 450 HV_{0.1}.

2. Dental components as claimed in claim 1, **characterized in that** the glass solder is applied by means of spray technology.

3. Dental components as claimed in claim 1 or claim 2, **characterized in that** the glass solder contains a material for coloration.

## Revendications

1. Composants dentaires constitués de matériaux céramiques de type oxyde qui sont à base de dioxyde de zirconium (ZrO₂), de matériaux céramiques mixtes de ce dernier, et/ou de matériaux vitrocéramiques, qui comportent des faces d'occlusion destinées à des dents naturelles ou artificielles opposées, et dont les surfaces ont subi un traitement, lesdits composants dentaires comportant un revêtement constitué d'une brasure de verre et étant soumis à une cuisson céramique puis étant pourvus en phase finale, sur la surface visible du composant dentaire (1) au niveau de la face d'occlusion, d'une couche (3) en une brasure de verre biocompatible constituée d'un système vitreux à base de dioxyde de silicium (SiO₂), pour ensuite subir une cuisson céramique, l'épaisseur de la couche vitrocéramique silicatée (3) étant inférieure à 20 micromètres, et le système vitreux présentant, après la cuisson céramique, une dureté inférieure à une fourchette allant de 400 à 450 HV_{0,1}.

2. Composants dentaires selon la revendication 1, **caractérisés en ce que** ladite brasure de verre est appliquée par une technique de pulvérisation.

3. Composants dentaires selon les revendications 1 ou 2, **caractérisés en ce que** ladite brasure de verre contient une matière destinée à la colorer.
